# EUROPEAN PATENT SPECIFICATION

(11) **EP 4 554 547 B1**
(45) Date of publication and mention of the grant of the patent: **18.03.2026**
(21) Application number: 23736775.0
(22) Date of filing: 12.07.2023
(51) Int. Cl.: A61K 8/34, A61K 8/365, A61K 8/60, A61K 8/73, A61Q 5/00, A61Q 5/06, A61K 8/04, A61Q 5/12, A61K 8/39

(54) **COSMETIC COMPOSITION FOR THE SEMI-PERMANENT DEFORMATION OF KERATIN FIBERS**
KOSMETISCHE ZUSAMMENSETZUNG ZUR SEMIPERMANENTEN VERFORMUNG VON KERATINFASERN
COMPOSITION COSMÉTIQUE POUR LA DÉFORMATION SEMI-PERMANENTE DE FIBRES DE KÉRATINE

(30) Priority: 13.07.2022 EP 22184654
(43) Date of publication of application: 21.05.2025
(73) Proprietor: Wella Germany GmbH, 64295 Darmstadt (DE)
(72) Inventor: HERMANN, David, 64295 DARMSTADT (DE); TELSER, Sonja, 64295 DARMSTADT (DE); SCHMENGER, Jürgen, 78315 RADOLFZELL (DE)
(74) Representative: Bandpay & Greuter
(86) International application number: PCT/EP2023/069375
(87) International publication number: WO 2024/013260

(56) References cited:
- EP-A1- 3 973 944
- WO-A1-2022/063917
- US-A1- 2016 296 449
- WING LI ET AL: "ELCO-CARE(TM) Diutan Gum (Sphingomonas Ferment Extract) in Hair Care Formulations", IP.COM, IP.COM INC., WEST HENRIETTA, NY, US, 31 March 2020 (2020-03-31), XP013186135, ISSN: 1533-0001

## Description

### Field

The presently claimed invention relates to a cosmetic composition for the semi-permanent deformation of hair, a method and a use thereof. The cosmetic composition comprises at least one alpha-ketoacid, optionally in the form of its salts or its hydrates; at least one quaternary ammonium salt of an ethoxylated methyl glucose ether containing an average of 5 to 30 moles of ethylene oxide; diutan gum; at least one fatty alcohol; and at least one non-ionic surfactant.

### Background

In replacement of conventional methods for the permanent deformation of hair shaping, method for the semi-permanent deformation of hair comprising alpha-ketoacids are known. See for example, PCT application WO 2014/072491 A1 published on 15 May 2014; European application EP 3973944 A1 and EP 3973945 A1 published on 30 March 2022, which relate to compositions, processes and kits for semi-permanent straightening keratin fibers; PCT application WO 2022063917 A1 published on 31 March 2022, which relates to a permanent hair shaping composition; the application US 2016296449 A1 published on 13 October 2016, which relates to a semi-permanent hair straightening composition and method; and the article from Wing Li et al. entitled "KELCO-CARE™ Diutan Gum (Sphingomonas Ferment Extract) in Hair Care Formulations" and published on 31 March 2020.

Usually, in order to achieve satisfactory benefits in terms of straightening and anti-frizziness, complex formulations and chemical processes are used. Even though a satisfactory straightening and anti-frizziness may be achieved with known compositions, these compositions may impair hair shine and manageability. These compositions may also impart a stinging and itching sensation, after carrying out the flat-iron treatment. These compositions may also leave unwanted residues on hair. These compositions may also have a significant damage profile. In order to compensate these drawbacks and therefore to achieve consumer acceptance, additional compounds are usually incorporated to the compositions, which complexity them further. Various aspects including the incorporation of alpha-ketoacids at a significant amount, the acidic pH of the composition, the incorporation of additional compounds, etc. are associated with additional drawbacks, as they impair the sustainability of the composition and they also alter the hairdresser and/or consumer's experience during the flat-iron treatment, because of the generation of a significant amount of fume.

There is therefore the need for providing a cosmetic composition, which is suitable for the semi-permanent deformation of keratin fibres, particularly human hair. There is also the need for providing a composition, which may be used in the so-called *"Brazilian hair straightening method".* There is also the need for providing a composition, which has a broad range of activity, from eliminating hair frizz to straightening curls and waves. There is also the need for providing a composition, which is suitable for the semi-permanent deformation of hair, while providing superior shine to hair. There is also the need for providing a composition, which is suitable for the semi-permanent deformation of hair, while providing superior manageability to hair. There is also the need for providing a composition, which is suitable for the semi-permanent deformation of hair, while providing superior conditioning to hair. There is also the need for providing a composition, which is suitable for the semi-permanent deformation of hair, while imparting a reduced stinging and itching sensation or even preventing any stinging and itching sensation. There is also the need for providing a composition, which is suitable for the semi-permanent deformation of hair, while having a very low damage profile or even a no damage profile. There is also the need for providing a composition, which is suitable for the semi-permanent deformation of hair, while having a satisfactory sustainability, particularly by being an eco-ethical formulation.

Hence, there is a need for providing a composition, which is suitable for the semi-permanent deformation of hair, that imparts the benefits of hair compositions to the hair that are conventionally used for semi-permanent deformation such as of hair shine, hair manageability, hair conditioning and damage profile, while generating a minimal amount of fume during the application of the hair composition.

Accordingly, it is an object of the presently claimed invention to provide a composition, which is suitable for the semi-permanent deformation of hair, that imparts of hair compositions to the hair that are conventionally used for semi-permanent deformation such as of hair shine, hair manageability, hair conditioning and damage profile, while generating a minimal amount of fume during the application of the hair composition.

### Summary

Surprisingly, it was found that the combination of compounds including the quaternary ammonium salt of an ethoxylated methyl glucose ether containing an average of 5 to 30 moles of ethylene oxide and the diutan gum, particularly the combination of lauryl methyl gluceth-10 hydroxypropyldimonium chloride and diutan gum, in a gel network system obtained from the mixture of the fatty alcohol and the non-ionic surfactant, allows for maintaining the straightening performance provided by the alpha-ketoacids, without compromising the expected benefits, particularly in terms of hair shine, hair manageability, hair conditioning and damage profile and leads to the generation of less fume upon use in flat-iron treatments. Without wishing to be bound by any theory, the inventors believe that the satisfactory performance of the composition may be due to the physical and/or chemical interactions of both compounds. On one hand, upon application on hair, both compounds may form hydrogen bonds between each other (physical interactions). On the other hand, upon further application of heat, particularly using a heating device, both compounds may further form covalent bonds between each other (chemical interaction).

Hence, in a first aspect, the presently claimed invention relates to a cosmetic composition for the semi-permanent deformation of keratin fibres, preferably human hair, comprising:
(a) at least one alpha-ketoacid, optionally in the form of its salts or its hydrates;
(b) at least one quaternary ammonium salt of an ethoxylated methyl glucose ether containing an average of 5 to 30 moles of ethylene oxide;
(c) diutan gum;
(d) at least one fatty alcohol; and
(e) at least one non-ionic surfactant, which is different from component (d).

In a preferred embodiment, the at least one alpha-ketoacid is selected from the group consisting of glyoxylic acid, pyruvic acid and 2-ketobutyric acid; preferably the alpha-ketoacid is glyoxylic acid.

In a preferred embodiment, the at least one quaternary ammonium salt of an ethoxylated methyl glucose ether containing an average of 5 to 30 moles of ethylene oxide is lauryl methyl gluceth-10 hydroxypropyldimonium chloride.

In a preferred embodiment, the at least one fatty alcohol is selected from the group consisting of linear or branched C14 to C30 fatty alcohols; more preferably from the group consisting of linear C₁₄ to C₃₀ fatty alcohols; even more preferably from the group consisting of cetyl alcohol, stearyl alcohol, cetearyl alcohol and behenyl alcohol.

In a preferred embodiment, the at least one non-ionic surfactant is selected from the group consisting of polyoxyethylene C₈ to C₃₀ alkyl ethers; preferably from the group consisting of polyoxyethylene C₈ to C₃₀ alkyl ethers having at least 2 ethylene oxide units, or at least 5 ethylene oxide units; more preferably from the group consisting of polyoxyethylene C₈ to C₃₀ alkyl ethers having from 10 to 50 ethylene oxide units; more preferably from the group consisting of polyoxyethylene C₈ to C₃₀ alkyl ethers having from 15 to 30 ethylene oxide units; even more preferably from the group consisting of ceteareth-25 and steareth-20.

In a preferred embodiment, the at least one alpha-ketoacid is present in an amount in the range of ≥ 3.0 to ≤ 25.0 wt.-%, preferably in the range of ≥ 5.0 wt.-% to ≤ 22.0 wt.-%, more preferably in the range of ≥ 10.0 to ≤ 19.0 wt.-%, related to the total weight of the composition; the at least one quaternary ammonium salt of an ethoxylated methyl glucose ether is present in an amount in the range of ≥ 0.1 to ≤ 10.0 wt.-%, more preferably in the range of ≥ 0.1 to ≤ 5 wt.-%, even more preferably in the range of ≥ 0.5 to ≤ 3 wt.-%, related to the total weight of the composition; diutan gum is present in an amount in the range of ≥ 0.01 to ≤ 1.0 wt.-%, preferably in the range of ≥ 0.01 to ≤ 0.5 wt.-%, more preferably in the range of ≥ 0.01 to ≤ 0.3 wt.-%, related to the total weight of the composition; the at least one fatty alcohol is present in an amount in the range of ≥ 2.5 to ≤ 20.0 wt.-%, preferably in the range of ≥ 3 to ≤ 15 wt.-%, more preferably in the range of ≥ 4 to ≤ 10 wt.-%, related to the total weight of the composition; and/or the at least one non-ionic surfactant is present in an amount in the range of ≥ 0.2 to ≤ 5.0 wt.-%, more preferably in the range of ≥ 0.5 to ≤ 4 wt.-%, even more preferably in the range of ≥ 1.0 to ≤ 2.5 wt.-%, related to the total weight of the composition.

In a preferred embodiment, the composition has a pH of 4.0 or less, preferably in the range of ≥ 1 to ≤ 3.5, more preferably in the range of ≥ 1.5 to ≤ 3.0.

In a preferred embodiment, the composition has a viscosity in the range of ≥ 100 to ≤ 2,000 mPa.s, preferably in the range of ≥ 200 to ≤ 1,500 mPa.s, more preferably in the range of ≥ 500 to ≤ 1,000 mPa.s determined according to the method of the description.

In a preferred embodiment, the composition comprises the component (d) to the component (e) in a weight ratio in the range from ≥ 10.0:0.5 to ≤ 10:4.0, more preferably in the range of ≥ 10.0:1.0 to ≤ 10.0:3.5; even more preferably in the range of ≥ 10.0:1.5 to ≤ 10.0:3.0.

In a preferred embodiment, the component (d) and the component (e) are comprised in the composition, in part or all, in a gel network system.

In a preferred embodiment, the composition is a leave-on composition or a rinse-off composition; preferably a rinse-off composition.

In a second aspect, the presently claimed invention relates to a method for the semi-permanent deformation of hair, comprising at least the steps of:
(A) providing a cosmetic composition, as defined above;
(B) applying the cosmetic composition onto the hair; and
(C) applying heat onto hair using a heating device.

In a preferred embodiment, in step (C), the hair is heated to a temperature in the range of ≥ 150 to ≤ 250 °C, preferably in the range of ≥ 170 to ≤ 230 °C, more preferably in the range of ≥ 185 to ≤ 220°C.

In a preferred embodiment, in step (C), the heating device is a heating iron; preferably the heating iron is selected from the group consisting of a straightening flat iron or a crimping iron.

In a third aspect, the presently claimed invention relates to the use of the cosmetic composition, as defined above, for the semi-permanent deformation of hair.

### Description of the figures

Figures 1a and 1b represent photographs using scanning electronic microscopy (SEM) of samples of untreated hair (control), respectively at magnification 2.500x and 10.000x.
Figures 2a and 2b represents photographs using SEM of samples treated with the composition of the presently claimed invention, respectively at magnification 2.500x and 10.000x.

### Detailed description

Before the composition of the presently claimed invention and formulations of the presently claimed invention are described, it is to be understood that this invention is not limited to particular compositions and formulations described, since such compositions and formulation may, of course, vary. It is also to be understood that the terminology used herein is not intended to be limiting, since the scope of the presently claimed invention will be limited only by the appended claims.

If hereinafter a group is defined to comprise at least a certain number of embodiments, this is meant to also encompass a group which preferably consists of these embodiments only. Furthermore, the terms 'first', 'second', 'third' or 'a', 'b', 'c', etc. and the like in the description and in the claims, are used for distinguishing between similar elements and not necessarily for describing a sequential or chronological order. It is to be understood that the terms so used are interchangeable under appropriate circumstances and that the embodiments of the presently claimed invention described herein are capable of operation in other sequences than described or illustrated herein. In case the terms 'first', 'second', 'third' or '(A)', '(B)' and '(C)' or '(a)', '(b)', '(c)', '(d)', 'i', 'ii' etc. relate to steps of a method or use or assay there is no time or time interval coherence between the steps, that is, the steps may be carried out simultaneously or there may be time intervals of seconds, minutes, hours, days, weeks, months or even years between such steps, unless otherwise indicated in the application as set forth herein above or below.

Furthermore, the ranges defined throughout the specification include the end values as well, *i.e.,* a range of 1 to 10 implies that both 1 and 10 are included in the range. For the avoidance of doubt, applicant shall be entitled to any equivalents according to applicable law.

In the following passages, different aspects of the presently claimed invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to 'one embodiment' or 'an embodiment' means that a particular feature, structure or characteristic described in connection with the embodiment is included in at least one embodiment of the presently claimed invention. Thus, appearances of the phrases 'in one embodiment' or 'in an embodiment' in various places throughout this specification are not necessarily all referring to the same embodiment, but may refer to the same embodiment. Further, as used in the following, the terms "preferably", "more preferably", "even more preferably", "most preferably" and "in particular" or similar terms are used in conjunction with optional features, without restricting alternative possibilities. Thus, features introduced by these terms are optional features and are not intended to restrict the scope of the claims in any way.

Furthermore, the particular features, structures or characteristics may be combined in any suitable manner, as would be apparent to a person skilled in the art from this disclosure, in one or more embodiments. Furthermore, while some embodiments described herein include some, but not other features included in other embodiments, combinations of features of different embodiments are meant to be within the scope of the presently claimed invention, and form different embodiments, as would be understood by those in the art. For example, in the appended claims, any of the claimed embodiments can be used in any combination.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

As used herein the term *"hair"* may be *"living", i.e.,* on a living body, or may be *"non-living", i.e.,* in a wig, hairpiece or other aggregation of non-living keratinous fibres. Mammalian, preferably human hair is preferred. However, wool, fur and other keratin fibres (keratin-containing fibres) are suitable substrates for the composition of the presently claimed invention.

By *"a cosmetic composition for the semi-permanent deformation of keratin fibres"* is meant a hair composition, which is suitable for changing the waviness and frizziness of hair, albeit not permanently. Such composition is referred hereinafter as *"the composition"* or *"the composition of the presently claimed invention",* unless otherwise specified. The composition of the presently claimed invention may be used for straightening, relaxing and/or smoothing keratin fibres, preferably human hair. In contrast, the composition of the presently claimed invention is not suitable for use as a permanent shaping composition *i.e.,* a cosmetic composition for the permanent deformation (*i.e.,* waving) of keratin fibres, preferably human hair.

By *"substantially free of"* is meant a composition comprising 0.1 wt.-% or less, preferably 0.01 wt.-% or less, more preferably 0 wt.-%, of a compound, related to the total weight of the composition.

All percentages are by weight of the composition of the presently claimed invention, *i.e.,* of the ready-to-use composition, which is the composition to be applied on hair, unless otherwise specified. Also ratios are weight ratios unless specifically stated otherwise.

### Cosmetic composition

In a first aspect, the presently claimed invention relates to a cosmetic composition for the semi-permanent deformation of keratin fibres, preferably human hair, comprising: (a) at least one alpha-ketoacid, optionally in the form of its salts or its hydrates; (b) at least one quaternary ammonium salt of an ethoxylated methyl glucose ether containing an average of 5 to 30 moles of ethylene oxide; (c) diutan gum; (d) at least one fatty alcohol; and (e) at least one non-ionic surfactant.

### Alpha-ketoacids

The composition comprises at least one alpha-ketoacid, optionally in the form of its salts or its hydrates (component (a)).

In a preferred embodiment, the at least one alpha-ketoacid has the formula (I) below:

R-CO-COOH (I)

wherein R represents a hydrogen or a linear or branched, unsubstituted C₁-C₆ alkyl.

In a preferred embodiment, the at least one alpha-ketoacid is present in the composition in the free form, its hydrate forms, its salt forms; more preferably in the free form or its hydrate forms.

In a preferred embodiment, the salt of the at least one alpha-ketoacid is selected from the group consisting of the salts of alkali metals and the salts of alkaline-earth metals; preferably the salt of the at least one alpha-ketoacid is a sodium salt.

In a preferred embodiment, the at least one alpha-ketoacid is selected from the group consisting of glyoxylic acid, pyruvic acid and 2-ketobutyric acid, optionally in the form of their hydrates or their salts; preferably the at least one alpha-ketoacid is glyoxylic acid, optionally in the form of its hydrates.

In a preferred embodiment, the component (a) is present in an amount in the range of ≥ 3.0 to ≤ 25.0 wt.-%, more preferably in the range of ≥ 5.0 wt.-% to ≤ 22.0 wt.-%, even more preferably in the range of ≥ 10.0 to ≤ 19.0 wt.-%, related to the total weight of the composition.

### Quaternary ammonium salt of an ethoxylated methyl glucose ether

The composition comprises at least one quaternary ammonium salt of an ethoxylated methyl glucose ether containing an average of 5 to 30 moles of ethylene oxide (component (b)).

In a preferred embodiment, the at least one quaternary ammonium salt of an ethoxylated methyl glucose ether containing an average of 5 to 30 moles of ethylene oxide is lauryl methyl gluceth-10 hydroxypropyldimonium chloride.

Lauryl methyl gluceth-10 hydroxypropyldimonium chloride is commercially available under the tradename Glucquat^{™} 125 Humectant from Lubrizol.

In a preferred embodiment, the component (b) is present in an amount in the range of ≥ 0.1 to ≤ 10 wt.-%, more preferably in the range of ≥ 0.1 to ≤ 5 wt.-%, even more preferably in the range of ≥ 0.5 to ≤ 3 wt.-%, related to the total weight of the composition.

### Diutan gum

The composition comprises diutan gum (component (c)). Diutan gum is an anionic polysaccharide. It is a thickener and/or a rheology modifier, which is added in an amount sufficient to provide the composition with a viscosity so that it can be readily applied to the hair without unduly dripping off the hair and causing mess.

In a preferred embodiment, diutan gum has a weight average molecular weight in the range of ≥ 2.8 million Daltons to ≤ 5.2 million Daltons.

Diutan gum is commercially available under the tradename Kelco-Care^{™} Diutan Gum from CP Kelco France or Lubrizol. Diutan gum is a water-soluble polysaccharide, which may be produced by microbial fermentation, particularly from *Sphingomonas.* Diutan gum is therefore a Sphingomonas ferment extract. Diutan gum is composed by six sugar units of d-glucose, d-glucuronic acid, d-glucose (with 2 I-rhamnose in the side chain) and I-rhamnoses, forming a linear backbone with a repeating side chain.

In a preferred embodiment, diutan gum is present in an amount in the range of ≥ 0.01 to ≤ 1 wt.-%, more preferably in the range of ≥ 0.01 to ≤ 0.5 wt.-%, even more preferably in the range of ≥ 0.01 to ≤ 0.3 wt.-%, related to the total weight of the composition.

### Fatty alcohols

The composition comprises at least one fatty alcohol (component (d)). By *"fatty alcohol"* is meant a non-alkoxylated, saturated or unsaturated, linear or branched, alcohol having from 14 to 30 carbon atoms.

In a preferred embodiment, the at least one fatty alcohol is selected from the group consisting of linear C₁₄ to C₃₀ fatty alcohols; more preferably from the group consisting of cetyl alcohol, stearyl alcohol, cetearyl alcohol and behenyl alcohol. In a most preferred embodiment, the fatty alcohol is cetearyl alcohol.

In a preferred embodiment, the component (d) is present in an amount in the range of ≥ 2.5 to ≤ 20 wt.-%, preferably in the range of ≥ 3 to ≤ 15 wt.-%, more preferably in the range of ≥ 4 to ≤ 10 wt.-%, related to the total weight of the composition. The amount of the fatty alcohol described hereinbefore can account for up to 100 wt.-% (or 100 wt.-%) of the total amount of the at least one non-ionic surfactant in the composition.

### Non-ionic surfactants

The composition comprises at least one non-ionic surfactant (component (e)). By *"non-ionic surfactant"* is meant any non-ionic surfactant other than the fatty alcohol.

In a preferred embodiment, the at least one non-ionic surfactant is selected from the group consisting of non-ionic surfactants comprising one or more polyethylene oxide chain including the following compounds: polyoxyethylene alkyl ethers, polyethylene glycol fatty acid esters, polyoxyethylene castor oil, polyoxyethylene hydrogenated castor oil, polyoxyethylene fatty amides and their monoethanolamine and diethanolamine derivatives and polyethoxylated fatty amines. More preferably, the at least one non-ionic surfactant is selected from the group consisting of polyoxyethylene C₈ to C₃₀ alkyl ethers; even more preferably from the group consisting of polyoxyethylene C₈ to C₃₀ alkyl ethers having at least 2 ethylene oxide units, or at least 5 ethylene oxide units; yet more preferably from the group consisting of polyoxyethylene C₈ to C₃₀ alkyl ethers having from 10 to 50 ethylene oxide units; most preferably from the group consisting of polyoxyethylene C₈ to C₃₀ alkyl ethers having from 15 to 30 ethylene oxide units; in particular from the group consisting of ceteareth-25 and steareth-20. Steareth-20 is especially preferred.

In a preferred embodiment, the component (e) is present in amount in the range of ≥ 0.2 to ≤ 5 wt.-%, more preferably in the range of ≥ 0.5 to ≤ 4 wt.-%, even more preferably in the range of ≥ 1.0 to ≤ 2.5 wt.-%, related to the total weight of the composition. The amount of the non-ionic surfactant described hereinbefore can account for up to 100 wt.-% (or 100 wt.-%) of the total amount of non-ionic surfactant in the composition.

### Weight ratio fatty alcohol : non-ionic surfactant

In a preferred embodiment, the composition comprises the component (d) to the component (e) in a weight ratio in the range of ≥ 10.0:0.5 to ≤ 10.0:4.0, more preferably in the range of ≥ 10.0:1.0 to ≤ 10.0:3.5; even more preferably in the range of ≥ 10.0:1.5 to ≤ 10.0:3.0.

### Gel network system

In a preferred embodiment, the at least one fatty alcohol and the non-ionic surfactant are comprised in the composition, in part or all, in a gel network system.

In a preferred embodiment, the composition comprises a total amount of the component (d) and the component (d) as described herein above in the range of ≥ 2.7 to ≤ 25 wt.-%, preferably in the range of ≥ 3.5 to ≤ 19 wt.-%, more preferably in the range of ≤ 5.0 to ≥ 12.5 wt.-%, related to the total weight of the composition.

In a preferred embodiment, the formation of the gel network involves heating a dispersion of the at least one fatty alcohol in water with the surfactant to a temperature above 80 °C. During the mixing process, the fatty alcohol melts, allowing the surfactant to partition into the fatty alcohol droplets. The surfactant brings water along with it into the at least one fatty alcohol. This changes the isotropic fatty alcohol drops into liquid crystalline phase drops. When the mixture is cooled below the chain melt temperature the liquid crystal phase is converted into a solid crystalline gel network. The gel network may particularly contribute to reduce, or even eliminate, the odour of the compounds comprising a mercapto-functional group.

In a preferred embodiment, from ≥ 50 to ≤ 100 wt.-%, more preferably from ≥ 70 to ≤ 100 wt.-%, even more preferably from ≥ 90 to ≤ 100 wt.-%, most preferably 100wt.-% of the component (d) is comprised within the gel network. In a preferred embodiment, from ≥ 50 to ≤ 100 wt.-%, more preferably from ≥ 70 to ≤ 100 wt.-%, even more preferably from ≥ 90 to ≤ 100 wt.-%, most preferably 100 wt.-% of the component (e) is comprised within the gel network.

### Cosmetically acceptable carrier

In a preferred embodiment, the composition comprises at least one cosmetically acceptable carrier or solvent.

In a preferred embodiment, the solvent is an aqueous carrier; more preferably an aqueous carrier selected from the group consisting of water and water-soluble polyhydric alcohols; even more preferably an aqueous carrier selected from the group consisting of water and glycerin; most preferably the aqueous carrier is water or alternatively a mixture of water and glycerin.

Water-soluble polyhydric alcohols may have two or more hydroxyl groups in the molecule. Polyhydric alcohols may be selected from the group consisting of dihydric alcohols such as ethylene glycol, 1,2- or 1,3-propanediol, 1,2-pentanediol; further, trimethylene glycol, 1,2-butylene glycol, 1,3-butylene glycol, 1,4-butylene glycol, tetramethylene glycol, 2,3-butylene glycol, pentamethylene glycol, 2-butene-1,4-diol, hexylene glycol, octylene glycol; trihydric alcohols such as glycerol, trimethylol propane, 1,2,6-hexanetriol and the like; tetrahydric alcohols such as penthaerythritol; pentahydric alcohols such as xylytol, etc.; hexahydric alcohols such as sorbitol, mannitol; polyhydric alcohol polymers such as diethylene glycol, dipropylene glycol, polyethylene glycol, polypropylene glycol, tetraethylene glycol, diglycerol, polyethylene glycol, triglycerol, tetraglycerol, polyglycerol; dihydric alcohol alkyl ethers such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether; dihydric alcohol alkyl ethers such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether; dihydric alcohol etheresters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate and propylene glycol monophenyl ether acetate. In a particularly preferred embodiment, the water-soluble polyhydric alcohol is selected from the group consisting of propylene glycol and glycerol.

In a preferred embodiment, the cosmetically acceptable carrier is present in an amount of ≥ 55.0 wt.-%, more preferably of ≥ 60.0 wt.-%, even more preferably of ≥ 65.0 wt.-%, related to the total weight of the composition. In a preferred embodiment, the cosmetically acceptable carrier is present in an amount in the range of ≥ 55.0 to ≤ 90.0 wt.-%, more preferably in the range of ≥ 60.0 to ≤ 85.0 wt.-%, even more preferably in the range of ≥ 65.0 to ≤ 80.0 wt.-%, related to the total weight of the composition.

The composition may comprise water as the sole cosmetically acceptable carrier. In this embodiment, the composition is substantially free of water-soluble polyhydric alcohols. In a preferred embodiment, water is present in an amount of ≥ 55.0 wt.-%, more preferably of ≥ 60.0 wt.-%, even more preferably of ≥ 65.0 wt.-%, related to the total weight of the composition. In a preferred embodiment, water is present in an amount in the range of ≥ 55.0 to ≤ 90.0 wt.-%, preferably in the range of ≥ 60.0 to ≤ 85.0 wt.-%, more preferably in the range of ≤ 65.0 to ≤ 80.0 wt.-%, related to the total weight of the composition.

Alternatively, the composition may comprise water as the main cosmetically acceptable carrier and at least one water-soluble polyhydric alcohol as a secondary cosmetically acceptable carrier. In a preferred embodiment, water is present in an amount of ≥ 50.0 wt.-%, preferably of ≥ 55.0 wt.-%, more preferably of ≥ 60.0 wt.-%, related to the total weight of the composition, and the remaining amount of the cosmetically acceptable carrier consists of the one water-soluble polyhydric alcohol. In a preferred embodiment, water is present in an amount in the range of ≥ 50.0 to ≤ 85.0 wt.-%, preferably in the range of ≥ 55.0 to ≤ 80.0 wt.- %, more preferably in the range of ≥ 60.0 to ≤ 75.0 wt.-%, related to the total weight of the composition, and the remaining amount of the cosmetically acceptable carrier consists of the one water-soluble polyhydric alcohol.

### Additional surfactants

In a preferred embodiment, the composition comprises at least one surfactant, other than the non-ionic surfactant, albeit in a low amount, e.g., in an amount in the range of ≥ 0.1 to ≤ 5.0 wt.-%. A suitable surfactant generally has a lipophilic chain length of from 8 to 30 carbon atoms and may be selected from anionic surfactants, amphoteric surfactants and cationic surfactants.

However, in an alternative preferred embodiment, the composition is substantially free of an additional surfactant, other than the non-ionic surfactant. The composition may be substantially free of an anionic surfactant. The composition may be substantially free of an amphoteric surfactant. The composition may be substantially free of a cationic surfactant.

### Additional thickeners and/or rheology modifiers (substantially free of)

In a preferred embodiment, the composition is substantially free of additional thickeners and/or rheology modifiers, other than diutan gum.

For example, the additional thickeners and/or rheology modifiers may be selected from the group consisting of xanthan gum, tara gum, guar, hydroxypropyl guar, scleroglucan, methylcellulose, ethyl cellulose, hydroxyethyl cellulose, carboxymethyl cellulose, hydroxypropylmethyl cellulose, microcrystalline cellulose, hydroxybutylmethyl cellulose, hydroxypropyl cellulose, hydroxyethyl ethyl cellulose, cetyl hydroxyethyl cellulose, N-vinylpyrollidone, Acrylates/Ceteth-20 Itaconate Copolymer, hydroxypropyl starch phosphate, polyethoxylated urethanes or polycarbamyl polyglycol ester such as PEG-150/Decyl/SMDI copolymer, PEG-150/Stearyl/SMDI copolymer, trihydroxystearin, acrylates copolymer or hydrophobically modified acrylate copolymers (such as Acrylates / Steareth-20 Methacrylate Copolymer), acrylates/steareth-20 methacrylate crosspolymer, acrylates/vinyl neodecanoate crosspolymer, acrylates/beheneth-25 methacrylate copolymer, acrylates/Clü-30 alkyl acrylate crosspolymer, and non-ionic amphophilic polymers comprising at least one fatty chain and at least one hydrophilic unit selected from polyether urethanes comprising at least one fatty chain; in particular xanthan gum.

### Cationic polymers (substantially free of)

In a preferred embodiment, the composition is substantially free of a cationic polymer, other than the quaternary ammonium salt of an ethoxylated methyl glucose ether.

### Reducing agent (substantially free of)

In a preferred embodiment, the composition is substantially free of a reducing agent, particularly a reducing agent comprising a mercapto-functional group. Reducing agents are compounds, which are suitable for reducing the disulfide bonds of the hair. Reducing agents are usually present in cosmetic compositions for the permanent deformation of keratin fibres. For example, the reducing agents may be selected from the group consisting of cysteamine, cysteine, thioglycolic acid and their salts thereof.

### Alkalizing agent (substantially free of)

In a preferred embodiment, the composition is substantially free of an alkalizing agent, other than pH adjusting agents. Alkalizing agents are usually present in cosmetic compositions for the permanent deformation of keratin fibres. For example, the alkalizing agent may be selected from the group consisting of ammonia and monoethanolamine.

### Colouring agent (substantially free of)

In a preferred embodiment, the composition is substantially free of a colouring agent. Colouring agents may be selected from the group consisting of direct dyes, pigments and oxidation dye precursor (including bases and couplers).

### Liquid Oils

In a preferred embodiment, the composition comprises at least one liquid oil.

For example, the liquid oil may be selected from the group consisting of avocado oil, tsubaki oil, turtle oil, macadamia nuts oil, corn oil, mink oil, olive oil, rape seed oil, yolk oil, sesame oil, parsic oil, wheat germ oil, sasanqua oil, castor oil, linseed oil, safflower oil, cotton seed oil, perilla oil, soybean oil, peanut oil, tea seed oil, kaya oil, rice bran oil, Chinese tung oil, Japanese tung oil, jojoba oil, germ oil, triglycerine, trioctanoic acid glycerine, triisopalmitic acid glycerine, liquid lanolin, hexyl laurate, liquid hydrocarbon esters such as fluid paraffin, mineral oil, isododecane, fatty acid oils, squalene, ester oils such as cetyl octanoate and isopropyl myristate.

If present, the at least one liquid oil is present in an amount in the range of ≥ 0.1 to ≤ 5.0 wt.-%, related to the total weight of the composition. Alternatively, the composition may be substantially free of a liquid oil. The absence of a liquid oil may help avoiding generating a significant amount of fume, when the hair is heated, for example by using a flat iron.

### Solid fats and waxes

In a preferred embodiment, the composition comprises at least one solid fat and wax.

For example, the solid fat and wax may be selected from the group consisting of cacao fat, coconut oil, horse fat, hardened coconut fat, palm oil, tallow, sheep fat, hardened tallow, palm kernel oil, lard, ox bone fat, wood wax kernel oil, hardened castor oil; lanolin, kapok wax, lanolin acetate, isopropyl lanolin fatty acid, reduced lanolin, hard lanolin, POE lanolin alcohol ether, POE lanolin alcohol acetate, POE cholesterol ether, lanolin fatty acid polyethylene glycol, POE hydrogenated lanolin alcohol ether; hydrocarbons, non-volatile hydrocarbons and solid hydrocarbon esters such as solid paraffin, vaseline, ozocerite, pristan, ceresin, petrolatum, microcrystalline wax; phospholipides, e.g., lecithins or ceramides, beeswax, apple wax, candelilla wax, cotton wax, carnauba wax, bayberry wax, insect wax, whale wax, montan wax, rice bran wax, kapok wax, cane wax, jojoba wax, and shellac wax. The wax is not a fatty alcohol.

If present, the at least one solid fat and wax is present in an amount in the range of ≥ 0.1 to ≤ 5.0 wt.-%, related to the total weight of the composition. Alternatively, the composition may be substantially free of a solid fat and wax. The absence of a solid fat and wax may help avoiding generating a significant amount of fume, when the hair is heated, for example by using a flat iron.

### Silicones

In a preferred embodiment, the composition comprises at least one silicone. Silicones may be volatile or non-volatile, and soluble or insoluble. By *"soluble"* is meant that the silicone is miscible with the aqueous carrier of the composition so as to form part of the same phase. By *"insoluble"* is meant that the silicone forms a separate, discontinuous phase from the aqueous carrier, such as in the form of an emulsion or a suspension of droplets of the silicone.

For example, the soluble silicone may be selected from the group consisting of silicone copolyols, such as dimethicone copolyols, e.g., polyether siloxane-modified polymers, such as polypropylene oxide, polyethylene oxide modified polydimethylsiloxane, wherein the level of ethylene and/or propylene oxide sufficient to allow solubility in the composition. The insoluble silicone may have a viscosity of from 1,000 to 2,000,000 mPa.s at 25°C, preferably from 10,000 to 1,800,000 mPa.s, more preferably from 100,000 to 1,500,000 mPa.s. The viscosity can be measured by means of a glass capillary viscometer as set forth in Dow Corning Gorparate Test Method CTM0004, July 20, 1970. Particularly, the insoluble silicone may be selected from the group consisting of polyalkyl siloxanes, polyaryl siloxanes, polyalkylaryl siloxanes, polyether siloxane copolymers, dimethylpolysiloxane containing terminal hydroxyl groups, methylphenyl polysiloxane containing terminal hydroxyl groups and mixtures thereof, low molecular weight oligomeric polydimethylsiloxane or cyclic polydimethylsiloxane, polyether siloxane copolymers, Silicone conditioning polymers that are specially preferred are CTFA: dimethicone, amodimethicone, dimethicone bisamino hydroxypropyl copolyol, bisamino PEG/PPG-41/3 aminoethyl PG-propyl dimethicone, dihydroxypolydimethylsiloxane (CTFA: dimethiconol). Also preferred are volatile silicones such as e.g., CTFA: dimethicone, dimethicone copolyol and cyclodimethicone.

If present, the at least one silicone is present in an amount in the range of ≥ 0.1 to ≤ 6.0 wt.-%, related to the total weight of the composition. Alternatively, the composition may be substantially free of silicones.

### Swelling agents

In a preferred embodiment, the composition comprises at least one swelling agent. So-called swelling agents and penetrating substances may be added to the hair shaping composition, examples being urea, melamine, ethers, e. g. dipropylene glycol monomethyl ether, 2-pyrrolidon, imidazolidin-2-one, 1-methyl-2-pyrrolidone; alkali or ammonium thiocyanate, polyvalent alcohols, isopropanol; preferably the swelling agent and penetrating substance is urea.

If present, the swelling agent is present in an amount in the range of ≥ 0.1 to ≤ 5.0 wt.- %, related to the total weight of the composition. Alternatively, the composition may be substantially free of swelling agents.

### Additives

The composition may further comprise usual and known additives.

For example, suitable additives are: alcohols such as ethanol, propanol, isopropanol; sugar alcohols such as sorbitol, maltitol, maltotriose, mannitol, sucrase, erythritol, glucose, fructose, starch sugar, maltose, xylytose, starch sugar reduced alcohol; preservatives such as benzyl alcohol, methyl paraben, propyl paraben and imidazolidinyl urea; antioxidants such as tocopheroles; sequestering agents such as disodium ethylenediamine tetraacetate, ethylenediamine-N,N'-disuccinic acid and its salts; salts such as sodium chloride, potassium chloride, ammonium chloride, sodium sulfate, potassium sulfate and ammonium sulfate salts such as sodium chloride, potassium chloride, ammonium chloride, sodium sulfate, potassium sulfate and ammonium sulfate; pearlescent aids such as ethylene glycol distearate and Mica; UV-filters and sunscreens, *e.g*., such as p-methoxy cinnamic acid isoamyl ester, lipophile cinnamic acid esters, salicylic acid esters, 4-amino benzoic acid derivatives or hydrophilic sulfonic acid derivatives of benzophenones or 3-benzyliden camphor; pH adjusting agents, such as citric acid, formic acid, acetic acid, lactic acid, pyruvic acid, sodium citrate, succinic acid, phosphoric acid, sodium hydroxide, sodium carbonate; opacifiers such as kaolin and titanium oxide; solubilizers; perfume oils; dyes; and amino acids and/or hydrolysed proteins.

If present, the alcohol is present in an amount in the range of ≥ 0.1 to ≤ 20 wt.-%; each of the sugar alcohols, salts, pearlescent aids and UV-filters and sunscreens is present in an amount in the range of ≥ 0.1 to ≤ 5.0 wt.-%; each of antioxidants, pH adjusting agents, sequestering agents, opacifiers, preservatives, perfume oils, solubilizers, amino acids and hydrolysed proteins and/or dyes is present in an amount in the range of ≥ 0.01 to ≤ 1.0 wt.-% of, related to the total weight of the composition.

### pH

In a preferred embodiment, the composition has a pH of 4.0 or less, more preferably in the range of ≥ 1.0 to ≤ 3.5, more preferably in the range of ≥ 1.5 to ≤ 3.0.

The pH of the composition may be adjusted using pH adjusting agents, including acidifying and/or basifying agents. The basifying agents may be selected from the group consisting of mineral alkaline agents, organic alkaline agents, and hydride alkaline agents. For example, suitable mineral alkaline agents may be selected from the group consisting of sodium hydroxide and potassium hydroxide; preferably the mineral alkaline agent is sodium hydroxide.

### Viscosity

In a preferred embodiment, the composition has a viscosity at 25°C in the range of ≥ 100 to ≤ 2,000 mPa.s, preferably in the range of ≥ 200 to ≤ 1,500 mPa.s, more preferably in the range of ≥ 500 to ≤ 1,000 mPa.s.

In a preferred embodiment, the viscosity is determined as follows: Viscosity measurements are carried out on a controlled stress rheometer of AR500, AR1000 or AR2000 type manufactured by TA Instruments, or equivalent instrument. A 6 cm flat acrylic cross hatched parallel plate geometry (TA item 518600.901) and a stainless-steel cross hatched base plate (TA item 570011.001) are used. The rheometer is prepared for flow measurements as per standard manufacturer procedure. The parallel plate geometry gap is set to 1,000 microns. The flow procedure is programmed to the rheometer with the following conditions: continuous stress ramp 0.1-300 Pa over 2 minutes at 25°C, including 250 measurement points in linear mode. The product is loaded into the geometry as per standard procedure. Shear stress value at 10 sec⁻¹ shear rate is obtained from the shear stress vs. shear rate curve, and the corresponding viscosity is calculated by dividing the obtained shear stress by 10.

### Application form

In one embodiment, the composition is a leave-on composition. By *"leave-on"* is meant a composition, which is not rinsed off after application onto hair, particularly before flat-ironing hair. In an alternative embodiment, the composition is a rinse-off composition. By *"rinse-off"* is meant a composition, which is rinsed off after application onto hair, particularly before flat-ironing hair. The composition is preferably a rinse-off composition.

The inventors have shown that, after application, the composition generates minimal fume when hair heated, for example by using a flat iron. This is advantageous, particularly by comparison with conventional cosmetic compositions used for the semi-permanent deformation of hair. Indeed, conventional cosmetic compositions may generate a significant amount of fume, when hair is heated, for example by using a flat iron. This is inconvenient for the hairdresser and/or the user, as it may irritate the eyes and mucous membranes, and generate an unwanted/unpleasant smell. This is particularly inconvenient in hair salons, whether several hairdressers may perform the treatment at the same time.

### Method for the semi-permanent deformation of hair

In a second aspect, the presently claimed invention relates to a method for the semi-permanent deformation of hair. This method comprises the following steps:
(A) providing a cosmetic composition, as described above;
(B) applying the cosmetic composition onto the hair;
(C) applying heat onto hair using a heating device.

The composition may be applied onto dry hair. The composition may be left on the hair at ambient temperature, preferably at a temperature in the range of ≥ 18 to ≤ 35°C. The time of contact of the composition with hair is preferably in the range of ≥ 10 to ≤ 60 min, more preferably in the range of ≥ 20 to ≤ 40 min.

The application of heat onto hair (*i.e.,* the heat treatment) preferably consists of heating hair to a temperature in the range of ≥ 150 to ≤ 250°C, preferably in the range of ≥ 170 to ≤ 230°C, more preferably in the range of ≥ 185 to ≤ 220°C.

When applying heat, the heating device is passes onto hair at least one time, preferably from 5 to 15 times, more preferably from 8 to 10 times. The heating device is preferably selected from known heating devices; more preferably the heating device is a heating iron, which is preferably selected from the group consisting of a straightening flat iron and a crimping iron. The heating iron usually comprises heating tongs, which are generally metallic heating tongs.

Prior to applying the composition of the presently claimed invention, the hair may be washed for example using a shampoo composition, and rinse with water. After rinsing and prior application of the composition of the presently claimed invention, the hair may be dried for example using a blow dryer.

Prior to applying the composition of the presently claimed invention, a pre-treatment composition may be applied to the hair.

A post-treatment composition may be applied to the hair, after applying heat. The post-treatment composition may be for example a conditioning composition.

### Use

In a third aspect, the presently claimed invention relates to the use of the composition described above, for the semi-permanent deformation of hair.

### Advantages

The presently claimed invention offers one or more of the following advantages:
- The composition provides a superior semi-permanent shaping performance;
- The composition provides hair manageability and hair conditioning;
- The composition does not impart any unpleasant stinging and itching sensation;
- The composition does not damage hair;
- The composition generates a minimal amount of fume upon application;
- The composition provides satisfactory consistency, rinsability, combability after wash (before flat ironing), combability with product, hair feel before flat ironing, hair feel after blow drying and/or straightening iron use;
- The composition is easy to manufacture because it contains only very few components; and
- The composition is not deposited on the hair.

### Examples

The presently claimed invention is illustrated in detail by non-restrictive working examples which follow. More particularly, the test methods specified hereinafter are part of the general disclosure of the application and are not restricted to the specific working examples.

### Materials

Glyoxylic acid: Glyoxo HighPure 50 from Weylchem
Lauryl methyl gluceth-10 hydroxypropyldimonium chloride: Glucquat^{™} 125 Humectant from Lubrizol
Diutan gum: Kelco-Care^{™} Diutan Gum from CP Kelco France or Lubrizol
Cetearyl alcohol
Steareth-20
Water
Glycerin
Sodium hydroxide

### Preparation of the cosmetic composition

The formation of the gel network involves heating a dispersion of the fatty alcohol in water with the non-ionic surfactant to a temperature above 80°C. During the mixing process, the fatty alcohol melts, allowing the surfactant to partition into the fatty alcohol droplets. The surfactant brings water along with it into the fatty alcohol. This changes the isotropic fatty alcohol drops into liquid crystalline phase drops. When the mixture is cooled below the chain melt temperature, the liquid crystal phase is converted into a solid crystalline gel network. Then, the other materials are added to the mixture obtained.

### Formulations

**Table 1**

| Materials (actives) | Formulations (weight percent) | | |
|---|---|---|---|
| | A | B | C |
| Glyoxylic acid | 16.00 | 12.00 | 12.00 |
| Cetearyl alcohol | 6.75 | 6.00 | 6.00 |
| Glycerin | 4.99 | - | - |
| Lauryl methyl gluceth-10 hydroxypropyldimonium chloride | 2.00 | 1.00 | 1.00 |
| Steareth-20 | 1.48 | 0.30 | 2.40 |
| Sodium hydroxide | 1.20 | - | - |
| Diutan gum | 0.15 | 0.05 | 0.05 |
| Water | Q.s. 100 | Q.s. 100 | Q.s. 100 |
| Ratio fatty alcohol/non-ionic surfactant | 10:2.19 | 10:0.5 | 10:4 |

### Method for the semi-permanent deformation of hair

The semi-permanent shaping method comprises the following steps:
(A) washing hair with a shampoo composition;
(B) Combing or brushing the hair;
(C) Towel drying the hair;
(D) Blowing dry the hair, for example for a period of 1 min;
(E) Combing or brushing the hair;
(F) Applying the cosmetic composition for the semi-permanent deformation onto hair;
(G) Combing or brushing the hair;
(H) Leaving the composition for a sufficient period of time for the deformation of hair, for example approximately 20 min;
(I) Rinsing-off the hair, for completely washing off the composition;
(J) Blowing dry the hair; and
(K) Dividing the hair in thin strands and pulling the flat iron through the strands from root to tip 6 to 8 times.

### Example 1: Half-head tests

The hair damage caused by the composition of the presently claimed invention (invention) was tested on 5 different models using the half-head test, using scanning electronic microscopy (SEM).

### Tested formulations

The composition (invention) has the following formula: 15.0 wt.-% glyoxylic acid, 6.75 wt.-% cetearyl alcohol, 4.96 wt.-% glycerin, 2.0 wt.-% Lauryl methyl gluceth-10 hydroxypropyldimonium chloride, 1.48 wt.-% steareth-20, 3.0 wt.-% sodium hydroxide, 0.15 wt.-% diutan gum, q.s. 100 wt.-% water.

The untreated hair was used as the control.

### Preparation of samples

Approximately 10 single hair fibres per sample were glued onto an alumina stub with conductive double-adhesive tape

### SEM protocol

The SEM examination was carried out using the following conditions:
- Instrument: Zeiss Ultra 55 from Mantis
- Mode: HV
- Acc. Voltage: 0/8 kV
- Fee working distance: 7 mm
- Tilt: 0°
- Detection: SE2
- Coating: uncoated
- Documentation: *.TIF

### Results

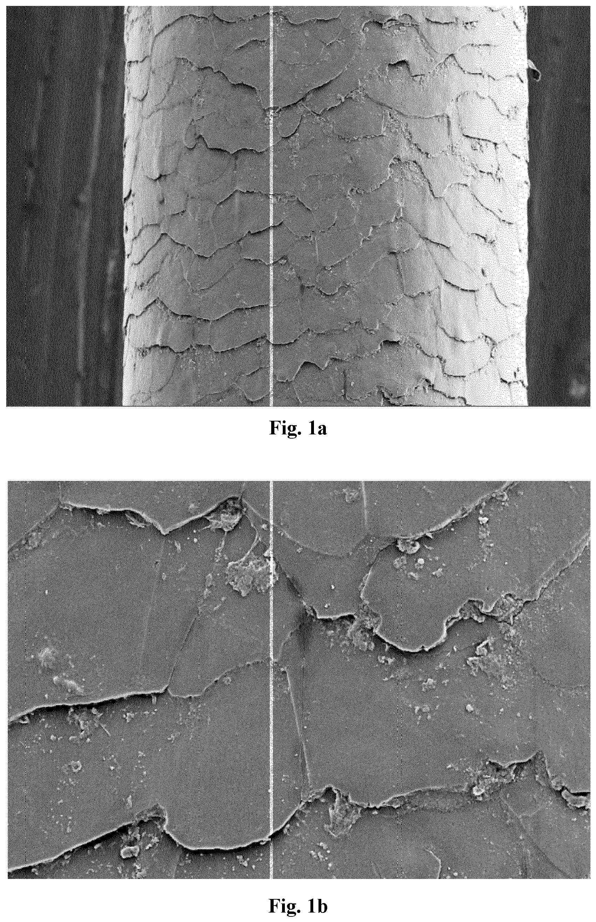

The untreated samples (control) only have from nearly no damages to small damages (see figures 1a/b for illustration).

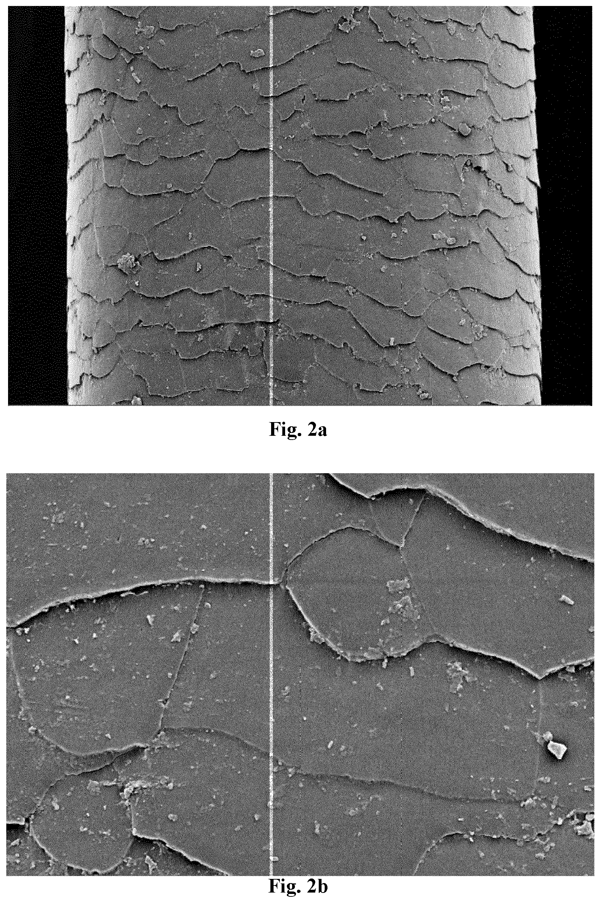

None of the five samples tested with the composition (invention) shows, compared to the untreated samples, any recognizable damage, corresponding to a 100% success. See figures 2a/b for illustration of an undamaged sample treated with the composition (invention).

Variations and modifications of the invention and further embodiments thereof, in addition to those described herein, will become apparent to those skilled in the art from the full contents of this document. The subject matter herein contains information, exemplification and guidance that can be adapted to the practice of this invention in its various embodiments and equivalents thereof. It is intended that the appended claims cover all such variations, modifications, embodiments and equivalents.

### Example 2: Comparative tests

The performance of the composition of the presently claimed invention (composition A) was compared with the performance of three comparative compositions (comparisons), being either free of diutan gum (composition 1), free of a quaternary ammonium salt of an ethoxylated methyl glucose ether containing an average of 5 to 30 moles of ethylene oxide (composition 2), or free of both compounds (composition 3).

### Tested formulations

The tested compositions have the following formulas:

**Table 2**

| Materials (actives) | Formulations (weight percent) | | | |
|---|---|---|---|---|
| | A | 1 | 2 | 3 |
| Glyoxylic acid | 16.00 | 16.00 | 16.00 | 16.00 |
| Cetearyl alcohol | 6.75 | 6.75 | 6.75 | 6.75 |
| Glycerin | 4.99 | 4.99 | 4.99 | 4.99 |
| Lauryl methyl gluceth-10 hydroxypropyldimonium chloride | 2.00 | 2.00 | - | - |
| Steareth-20 | 1.48 | 1.48 | 1.48 | 1.48 |
| Sodium hydroxide | 1.20 | 1.20 | 1.20 | 1.20 |
| Diutan gum | 0.15 | - | 0.15 | - |
| Water | Q.s. 100 | Q.s. 100 | Q.s. 100 | Q.s. 100 |

### Protocol

Hair stylists applied the compositions on mannequin hair and evaluated and compared their performance based on different properties, benefits and/or advantages.

The hair stylists evaluated the performance of each composition for each property, benefit or advantage using the following scale:

**Table 3**

| Scale | Performance |
|---|---|
| 1 | Poor |
| 2 | Fair |
| 3 | Good |
| 4 | Very good |
| 5 | Excellent |

### Results

The evaluation results by the hair stylists are compiled in the table below:

**Table 4**

| | Formulations (weight percent) | | | |
|---|---|---|---|---|
| | A | 1 | 2 | 3 |
| Consistency | 5 | 4.5 | 1 | 1 |
| Rinsability | 5 | 5 | 2 | 3 |
| Combability after wash (before flat ironing) | 5 | 5 | 1 | 2 |
| Combability with product | 5 | 4.5 | 2 | 2 |
| Hair feel wet before flat ironing | 5 | 5 | 1 | 2 |
| Hair feel after blow drying | 5 | 4 | 1 | 2 |
| Straightening iron use | 5 | 4 | 1 | 1 |

The results reported in table 4 demonstrate that the composition of the presently claimed invention provides, beyond expectation, an equal performance or an improved performance, depending on the property, benefit or advantage taken into consideration.

## Claims

1. A cosmetic composition for the semi-permanent deformation of keratin fibres, preferably human hair, comprising:
(a) at least one alpha-ketoacid, optionally in the form of its salts or its hydrates;
(b) at least one quaternary ammonium salt of an ethoxylated methyl glucose ether containing an average of 5 to 30 moles of ethylene oxide;
(c) diutan gum;
(d) at least one fatty alcohol being a non-alkoxylated, saturated or unsaturated, linear or branched, alcohol having from 14 to 30 carbon atoms; and
(e) at least one non-ionic surfactant, which is different from component (d).

2. The composition according to claim 1, wherein the at least one alpha-ketoacid is selected from the group consisting of glyoxylic acid, pyruvic acid and 2-ketobutyric acid; preferably the alpha-ketoacid is glyoxylic acid.

3. The composition according to any of the preceding claims, wherein the quaternary ammonium salt of an ethoxylated methyl glucose ether containing an average of 5 to 30 moles of ethylene oxide is lauryl methyl gluceth-10 hydroxypropyldimonium chloride.

4. The composition according to any preceding claims, wherein the at least one fatty alcohol is selected from the group consisting of linear C₁₄ to C₃₀ fatty alcohols; preferably from the group consisting of cetyl alcohol, stearyl alcohol, cetearyl alcohol and behenyl alcohol.

5. The composition according to any preceding claims, wherein the at least one non-ionic surfactant is selected from the group consisting of polyoxyethylene C₈ to C₃₀ alkyl ethers; preferably from the group consisting of polyoxyethylene C₈ to C₃₀ alkyl ethers having at least 2 ethylene oxide units, or at least 5 ethylene oxide units; more preferably from the group consisting of polyoxyethylene C₈ to C₃₀ alkyl ethers having from 10 to 50 ethylene oxide units; preferably from the group consisting of polyoxyethylene C₈ to C₃₀ alkyl ethers having from 15 to 30 ethylene oxide units; more preferably from the group consisting of ceteareth-25 and steareth-20.

6. The composition according to any preceding claims, wherein:
(a) the at least one alpha-ketoacid is present in an amount in the range of ≥ 3.0 to ≤ 25.0 wt.-%, preferably in the range of ≥ 5.0 wt.-% to ≤ 22.0 wt.-%, more preferably in the range of ≥ 10.0 to ≤ 19.0 wt.-%, related to the total weight of the composition;
(b) the at least one quaternary ammonium salt of an ethoxylated methyl glucose ether is present in an amount in the range of ≥ 0.1 to ≤ 10.0 wt.-%, more preferably in the range of ≥ 0.1 to ≤ 5.0 wt.-%, even more preferably in the range of ≥ 0.5 to ≤ 3.0 wt.-%, related to the total weight of the composition;
(c) diutan gum is present in an amount in the range of ≥ 0.01 to ≤ 1.0 wt.-%, preferably in the range of ≥ 0.01 to ≤ 0.5 wt.-%, more preferably in the range of ≥ 0.01 to ≤ 0.3 wt.-%, related to the total weight of the composition;
(d) the at least one fatty alcohol is present in an amount in the range of ≥ 2.5 to ≤ 20.0 wt.-%, preferably in the range of ≥ 3.0 to ≤ 15.0 wt.-%, more preferably in the range of ≥ 4.0 to ≤ 10.0 wt.-%, related to the total weight of the composition; and/or
(e) the at least one non-ionic surfactant is present in an amount in the range of ≥ 0.2 to ≤ 5.0 wt.-%, more preferably in the range of ≥ 0.5 to ≤ 4.0 wt.-%, even more preferably in the range of ≥ 1.0 to ≤ 2.5 wt.-%, related to the total weight of the composition.

7. The composition according to any of the preceding claims, wherein the composition has a pH of 4.0 or less, preferably in the range of ≥ 1 to ≤ 3.5, more preferably in the range of ≥ 1.5 to ≤ 3.0.

8. The composition according to any of the preceding claims, wherein the composition has a viscosity at 25 °C in the range of ≥ 100 to ≤ 2,000 mPa.s, preferably in the range of ≥ 200 to ≤ 1,500 mPa.s, more preferably in the range of ≥ 500 to ≤ 1,000 mPa.s determined according to the method of the description.

9. The composition according to any of the preceding claims, wherein the composition comprises the component (d) to the component (e) in a weight ratio in the range of ≥ 10.0:0.5 to ≤ 10.0:4.0, more preferably in the range of ≥ 10.0:1.0 to ≤ 10.0:3.5; even more preferably in the range of ≥ 10.0:1.5 to ≤ 10.0:3.0.

10. The composition according to any of the preceding claims, wherein the component (d) and the component (e) are comprised in the composition, in part or all, in a gel network system

11. The composition according to any preceding claims, wherein the composition is a leave-on composition or a rinse-off composition; preferably a rinse-off composition.

12. A method for the semi-permanent deformation of hair, comprising at least the steps of:
(A) providing a cosmetic composition, according to any of the preceding claims;
(B) applying the cosmetic composition onto the hair; and
(C) applying heat onto hair using a heating device.

13. The method according to claim 12, wherein, in step (C), the hair is heated to a temperature in the range of ≥ 150 to ≤ 250°C, preferably in the range of ≥ 170 to ≤ 230°C, more preferably in the range of ≥ 185 to ≤ 220°C.

14. The method according to claim 12 or 13, wherein, in step (C), the heating device is a heating iron; preferably the heating iron is selected from the group consisting of a straightening flat iron or a crimping iron.

15. The use of the cosmetic composition, according to any one of claims 1 to 11, for the semi-permanent deformation of hair.

## Patentansprüche

1. Kosmetikzusammensetzung zur halbdauerhaften Verformung von Keratinfasern, vorzugsweise von menschlichem Haar, umfassend:
(a) zumindest eine Alpha-Ketosäure, wahlweise in Form deren Salze oder deren Hydrate;
(b) zumindest ein quartäres Ammoniumsalz eines ethoxylierten Methylglukoseethers, enthaltend durchschnittlich 5 bis 30 Mol Ethylenoxid;
(c) Diutangummi;
(d) zumindest einen Fettalkohol, der ein nichtalkoxylierter, gesättigter oder ungesättigter, linearer oder verzweigter Alkohol mit 14 bis 30 Kohlenstoffatomen ist; und
(e) zumindest ein nichtionisches Tensid, das sich von Komponente (d) unterscheidet.

2. Zusammensetzung nach Anspruch 1, wobei die zumindest eine Alpha-Ketosäure aus der Gruppe ausgewählt ist, die aus Glyoxylsäure, Benztraubensäure und 2-Ketobuttersäure besteht; wobei die Alpha-Ketosäure vorzugsweise Glyoxylsäure ist.

3. Zusammensetzung nach einem der vorstehenden Ansprüche, wobei das quartäre Ammoniumsalz eines ethoxylierten Methylglukosethers, der im Durchschnitt 5 bis 30 Mol Ethylenoxid enthält, Laurylmethylgluceth-10-hydroxypropyldimoniumchlorid ist.

4. Zusammensetzung nach einem vorstehenden Anspruch, wobei der zumindest eine Fettalkohol aus der Gruppe ausgewählt ist, die aus linearen C₁₄- bis C₃₀-Fettalkoholen besteht; der vorzugsweise aus der Gruppe ausgewählt ist, die aus Cetylalkohol, Stearylalkohol, Cetearylalkohol und Behenylalkohol besteht.

5. Zusammensetzung nach einem vorstehenden Anspruch, wobei das zumindest eine nichtionische Tensid aus der Gruppe ausgewählt ist, die aus Polyoxyethylen-C₈- bis -C₃₀-alkylethern besteht; vorzugsweise aus der Gruppe, die aus Polyoxyethylen-C₈- bis -C₃₀-alkylethern mit zumindest 2 Ethylenoxideinheiten oder zumindest 5 Ethylenoxideinheiten besteht; stärker bevorzugt aus der Gruppe, die aus Polyoxyethylen-C₈- bis -C₃₀-alkylethern mit 10 bis 50 Ethylenoxideinheiten besteht; vorzugsweise aus der Gruppe, die aus Polyoxyethylen-C₈- bis -C₃₀-alkylethern mit 15 bis 30 Ethylenoxideinheiten besteht; stärker bevorzugt aus der Gruppe, die aus Ceteareth-25 und Steareth-20 besteht.

6. Zusammensetzung nach einem vorstehenden Anspruch, wobei:
(a) die zumindest eine Alpha-Ketosäure in einer Menge in dem Bereich von ≥ 3,0 bis ≤ 25,0 Gew.-%, vorzugsweise in dem Bereich von ≥ 5,0 Gew.-% bis ≤ 22,0 Gew.-% und stärker bevorzugt in dem Bereich von ≥ 10,0 bis ≤ 19,0 Gew.-% relativ zu dem Gesamtgewicht der Zusammensetzung vorhanden ist;
(b) das zumindest eine quartäre Ammoniumsalz eines ethoxylierten Methylglukoseethers in einer Menge in dem Bereich von ≥ 0,1 bis ≤ 10,0 Gew.-%, stärker bevorzugt in dem Bereich von ≥ 0,1 bis ≤ 5,0 Gew.-% und noch stärker bevorzugt in dem Bereich von ≥ 0,5 bis ≤ 3,0 Gew.-% relativ zu dem Gesamtgewicht der Zusammensetzung vorhanden ist;
(c) Diutangummi in einer Menge in dem Bereich von ≥ 0,01 bis ≤ 1,0 Gew.-%, vorzugsweise in dem Bereich von ≥ 0,01 bis ≤ 0,5 Gew.-% und stärker bevorzugt in dem Bereich von ≥ 0,01 bis ≤ 0,3 Gew.-% relativ zu dem Gesamtgewicht der Zusammensetzung vorhanden ist;
(d) der zumindest eine Fettalkohol in einer Menge in dem Bereich von ≥ 2,5 bis ≤ 20,0 Gew.-%, vorzugsweise in dem Bereich von ≥ 3,0 bis ≤ 15,0 Gew.-% und stärker bevorzugt in dem Bereich von ≥ 4,0 bis ≤ 10,0 Gew.-% relativ zu dem Gesamtgewicht der Zusammensetzung vorhanden ist; und/oder (e) das zumindest eine nichtionische Tensid in einer Menge in dem Bereich von ≥ 0,2 bis ≤ 5,0 Gew.-%, stärker bevorzugt in dem Bereich von ≥ 0,5 Gew.-% bis ≤ 4,0 Gew.-% und noch stärker bevorzugt in dem Bereich von ≥ 1,0 bis ≤ 2,5 Gew.-% relativ zu dem Gesamtgewicht der Zusammensetzung vorhanden ist.

7. Zusammensetzung nach einem der vorstehenden Ansprüche, wobei die Zusammensetzung einen pH-Wert von 4,0 oder weniger, vorzugsweise in dem Bereich von ≥ 1 bis ≤ 3,5 und stärker bevorzugt in dem Bereich von ≥ 1,5 bis ≤ 3,0 aufweist.

8. Zusammensetzung nach einem der vorstehenden Ansprüche, wobei die Zusammensetzung laut Bestimmung mithilfe des Verfahrens der Beschreibung eine Viskosität bei 25 °C in dem Bereich von ≥ 100 bis ≤ 2000 mPa.s, vorzugsweise in dem Bereich von ≥ 200 bis ≤ 1500 mPa.s und stärker bevorzugt in dem Bereich von ≥ 500 bis ≤ 1000 mPa.s aufweist.

9. Zusammensetzung nach einem der vorstehenden Ansprüche, wobei die Zusammensetzung Komponente (d) und Komponente (e) in einem Gewichtsverhältnis in dem Bereich von ≥ 10,0:0,5 bis ≤ 10,0:4,0, stärker bevorzugt in dem Bereich von ≥ 10,0:1,0 bis ≤ 10,0:3,5; noch stärker bevorzugt in dem Bereich von ≥ 10,0:1,5 bis ≤ 10,0:3,0 aufweist.

10. Zusammensetzung nach einem der vorstehenden Ansprüche, wobei Komponente (d) und Komponente (e) in der Zusammensetzung teilweise oder allesamt in einem Gelnetzwerksystem umfasst sind.

11. Zusammensetzung nach einem vorstehenden Anspruch, wobei die Zusammensetzung eine nicht auszuspülende Zusammensetzung oder eine auszuspülende Zusammensetzung ist; vorzugsweise eine auszuspülende Zusammensetzung ist.

12. Verfahren zum halbdauerhaften Verformen von Haar, das zumindest die Schritte umfasst:
(A) Bereitstellen einer Kosmetikzusammensetzung nach einem der vorstehenden Ansprüche;
(B) Auftragen der Kosmetikzusammensetzung auf das Haar; und
(C) Anwenden von Hitze auf das Haar mithilfe eines Erhitzungsgeräts.

13. Verfahren nach Anspruch 12, wobei in Schritt (C) das Haar auf eine Temperatur in dem Bereich von ≥ 150 bis ≤ 250°C, vorzugsweise in dem Bereich von ≥ 170 bis ≥ 230 °C und stärker bevorzugt in dem Bereich von ≥ 185 bis ≤ 220 °C erhitzt wird.

14. Verfahren nach Anspruch 12 oder 13, wobei in Schritt (C) das Erhitzungsgerät ein Heizeisen ist; wobei das Heizeisen aus der Gruppe ausgewählt ist, die aus einem Glätteisen oder einem Kreppeisen besteht.

15. Verwendung der Kosmetikzusammensetzung nach einem der Ansprüche 1 bis 11 zur halbdauerhaften Verformung von Haar.

## Revendications

1. Composition cosmétique pour la déformation semi-permanente de fibres kératiniques, préférentiellement de cheveux humains, comprenant :
(a) au moins un alpha-cétoacide, éventuellement sous la forme de ses sels ou de ses hydrates ;
(b) au moins un sel d'ammonium quaternaire d'un éther de méthyl-glucose éthoxylé contenant en moyenne 5 à 30 moles d'oxyde d'éthylène ;
(c) de la gomme diutane ;
(d) au moins un alcool gras qui est un alcool non alcoxylé, saturé ou insaturé, linéaire ou ramifié, ayant de 14 à 30 atomes de carbone ; et
(e) au moins un tensioactif non-ionique qui est différent du composant (d).

2. Composition selon la revendication 1, dans laquelle l'au moins un alpha-cétoacide est choisi dans le groupe consistant en l'acide glyoxylique, l'acide pyruvique et l'acide 2-cétobutyrique ; préférentiellement l'alpha-cétoacide est l'acide glyoxylique.

3. Composition selon l'une quelconque des revendications précédentes, dans laquelle le sel d'ammonium quaternaire d'un éther de méthyl-glucose éthoxylé contenant en moyenne 5 à 30 moles d'oxyde d'éthylène est le chlorure de lauryl méthyl gluceth-10 hydroxypropyldimonium.

4. Composition selon l'une quelconque des revendications précédentes, dans laquelle l'au moins un alcool gras est choisi dans le groupe consistant en les alcools gras linéaires en C₁₄ à C₃₀ ; préférentiellement dans le groupe consistant en l'alcool cétylique, l'alcool stéarylique, l'alcool cétéarylique et l'alcool béhénylique.

5. Composition selon l'une quelconque des revendications précédentes, dans laquelle l'au moins un tensioactif non-ionique est choisi dans le groupe consistant en les éthers alkyliques en C₈ à C₃₀ polyoxyéthylénés ; préférentiellement dans le groupe consistant en les éthers alkyliques en C₈ à C₃₀ polyoxyéthylénés ayant au moins 2 motifs oxyde d'éthylène, ou au moins 5 motifs oxyde d'éthylène ; très préférentiellement dans le groupe consistant en les éthers alkyliques en C₈ à C₃₀ polyoxyéthylénés ayant de 10 à 50 motifs oxyde d'éthylène ; préférentiellement dans le groupe consistant en les éthers alkyliques en C₈ à C₃₀ polyoxyéthylénés ayant de 15 à 30 motifs oxyde d'éthylène ; très préférentiellement dans le groupe consistant en le cétéareth-25 et le stéareth-20.

6. Composition selon l'une quelconque des revendications précédentes, dans laquelle:
(a) l'au moins un alpha-cétoacide est présent en une quantité située dans la plage allant de ≥ 3,0 à ≤ 25,0 % en poids, préférentiellement dans la plage allant de ≥ 5,0 % en poids à ≤ 22,0 % en poids, très préférentiellement dans la plage allant de ≥ 10,0 à ≤ 19,0 % en poids, par rapport au poids total de la composition ;
(b) l'au moins un sel d'ammonium quaternaire d'un éther de méthyl-glucose éthoxylé est présent en une quantité située dans la plage allant de ≥ 0,1 à ≤ 10,0 % en poids, très préférentiellement dans la plage allant de ≥ 0,1 à ≤ 5,0 % en poids, plus préférentiellement dans la plage allant de ≥ 0,5 à ≤ 3,0 % en poids, par rapport au poids total de la composition ;
(c) la gomme diutane est présente en une quantité située dans la plage allant de ≥ 0,01 à ≤ 1,0 % en poids, préférentiellement dans la plage allant de ≥ 0,01 à ≤ 0,5 % en poids, très préférentiellement dans la plage allant de ≥ 0,01 à ≤ 0,3 % en poids, par rapport au poids total de la composition ;
(d) l'au moins un alcool gras est présent en une quantité située dans la plage allant de ≥ 2,5 à ≤ 20,0 % en poids, préférentiellement dans la plage allant de ≥ 3,0 à ≤ 15,0 % en poids, très préférentiellement dans la plage allant de ≥ 4,0 à ≤ 10,0 % en poids, par rapport au poids total de la composition ; et/ou
(e) l'au moins un tensioactif non-ionique est présent en une quantité située dans la plage allant de ≥ 0,2 à ≤ 5,0 % en poids, très préférentiellement dans la plage allant de ≥ 0,5 à ≤ 4,0 % en poids, plus préférentiellement dans la plage allant de ≥ 1,0 à ≤ 2,5 % en poids, par rapport au poids total de la composition.

7. Composition selon l'une quelconque des revendications précédentes, laquelle composition a un pH de 4,0 ou moins, préférentiellement situé dans la plage allant de ≥ 1 à ≤ 3,5, très préférentiellement dans la plage allant de ≥ 1,5 à ≤ 3,0.

8. Composition selon l'une quelconque des revendications précédentes, laquelle composition a une viscosité à 25°C située dans la plage allant de ≥ 100 à ≤ 2 000 mPa.s, préférentiellement dans la plage allant de ≥ 200 à ≤ 1 500 mPa.s, très préférentiellement dans la plage allant de ≥ 500 à ≤ 1 000 mPa.s, déterminée conformément au procédé de la description.

9. Composition selon l'une quelconque des revendications précédentes, laquelle composition comprend le composant (d) et le composant (e) en un rapport en poids situé dans la plage allant de ≥ 10,0/0,5 à ≤ 10,0/4,0, très préférentiellement dans la plage allant de ≥ 10,0/1,0 à ≤ 10,0/3,5 ; plus préférentiellement dans la plage allant de ≥ 10,0/1,5 à ≤ 10,0/3,0.

10. Composition selon l'une quelconque des revendications précédentes, dans laquelle le composant (d) et le composant (e) sont compris dans la composition, en partie ou en totalité, dans un système de matrice de gel.

11. Composition selon l'une quelconque des revendications précédentes, laquelle composition est une composition sans rinçage ou une composition à rincer ; préférentiellement une composition à rincer.

12. Procédé pour la déformation semi-permanente des cheveux, comprenant au moins les étapes de :
(A) fourniture d'une composition cosmétique de l'une quelconque des revendications précédentes ;
(B) application de la composition cosmétique sur les cheveux ; et
(C) application de chaleur sur les cheveux au moyen d'un dispositif chauffant.

13. Procédé selon la revendication 12, dans lequel, dans l'étape (C), les cheveux sont chauffés à une température située dans la plage allant de ≥ 150 à ≤ 250°C, préférentiellement dans la plage allant de ≥ 170 à ≤ 230°C, très préférentiellement dans la plage allant de ≥ 185 à ≤ 220°C.

14. Procédé selon la revendication 12 ou 13, dans lequel, dans l'étape (C), le dispositif chauffant est un fer chauffant ; préférentiellement le fer chauffant est choisi dans le groupe consistant en un fer plat à lisser et un fer à friser.

15. Utilisation de la composition cosmétique de l'une quelconque des revendications 1 à 11 pour la déformation semi-permanente des cheveux.
